(19) **Europäisches Patentamt**

**European Patent Office**

**Office européen des brevets**

(11) **EP 1 268 850 B1**

(12) **FASCICULE DE BREVET EUROPEEN**

(45) Date de publication et mention
de la délivrance du brevet:
**29.03.2006 Bulletin 2006/13**

(51) Int Cl.:
***C12Q 1/37*** *(2006.01)*

(21) Numéro de dépôt: **01921512.8**

(86) Numéro de dépôt international:
**PCT/FR2001/001058**

(22) Date de dépôt: **06.04.2001**

(87) Numéro de publication internationale:
**WO 2001/077369 (18.10.2001 Gazette 2001/42)**

(54) **DETECTION DE PROTEASES OU PEPTIDASES PAR FLUORESCENCE**

FLUORESZENZ-VERFAHREN ZUM NACHWEIS VON PROTEASEN ODER PEPTIDASEN

FLUORESCENCE DETECTION OF PROTEASES OR PEPTIDASES

(84) Etats contractants désignés:
**AT BE CH CY DE DK ES FI FR GB GR IE IT LI LU MC NL PT SE TR**

(30) Priorité: **07.04.2000 FR 0004507**

(43) Date de publication de la demande:
**02.01.2003 Bulletin 2003/01**

(73) Titulaires:
• **INSTITUT NATIONAL DE LA SANTE ET DE LA RECHERCHE MEDICALE (INSERM)**
  **75013 Paris (FR)**
• **CENTRE NATIONAL DE
  LA RECHERCHE SCIENTIFIQUE (CNRS)**
  **75016 Paris (FR)**

(72) Inventeurs:
• **ROQUES, Bernard, Pierre**
  **F-75014 Paris (FR)**
• **LUCIANI, Nathalie**
  **F-75011 Paris (FR)**
• **FOURNIE-ZALUSKI, Marie-Claude**
  **F-75011 Paris (FR)**
• **DE ROCQUIGNY, Hugues**
  **F-78000 Versailles (FR)**

(74) Mandataire: **Bernasconi, Jean Raymond**
  **c/o Cabinet Lavoix,
  2, Place d'Estienne d'Orves
  75441 Paris Cedex 09 (FR)**

(56) Documents cités:
**DE-A- 4 313 232          FR-A- 2 745 576**

• **SASAKI, HIROSHI ET AL: "Photoinduced electron transfer in gramicidin S analogs" PEPT. SCI. (1999), 36TH, 355-356, XP001004613**
• **FAHNOE DOUGLASS C ET AL: "Disulfide bonds in big ET-1 are essential for the specific cleavage at the Trp21-Val22 bond by soluble endothelin converting enzyme-1 from baculovirus/insect cells." ARCHIVES OF BIOCHEMISTRY AND BIOPHYSICS, vol. 373, no. 2, 15 janvier 2000 (2000-01-15), pages 385-393, XP001004795 ISSN: 0003-9861**
• **FITZGERALD ET AL: "A continuous fluorometric assay for the feline immunodeficiency virus protease" ANALYTICAL BIOCHEMISTRY, ACADEMIC PRESS, SAN DIEGO, CA,US, vol. 54, 15 décembre 1997 (1997-12-15), pages 226-230, XP002160419 ISSN: 0003-2697**
• **VON GELDERN T W ET AL: "A FLUOROGENIC ASSAY FOR ENDOTHELIN-CONVERTING ENZYME" PEPTIDE RESEARCH, vol. 4, no. 1, 1991, pages 32-35, XP001004528 ISSN: 1040-5704**
• **JOHNSON GARY D ET AL: "Development of an internally quenched fluorescent substrate selective for endothelin-converting enzyme-1." ANALYTICAL BIOCHEMISTRY, vol. 286, no. 1, 1 novembre 2000 (2000-11-01), pages 112-118, XP002168332 ISSN: 0003-2697**
• **LUCIANI NATHALIE ET AL: "Highly sensitive and selective fluorescence assays for rapid screening of endothelin-converting enzyme inhibitors." BIOCHEMICAL JOURNAL, vol. 356, no. 3, 2001, pages 813-819, XP001025608 ISSN: 0264-6021**

EP 1 268 850 B1

- **ANNE CHRISTINE ET AL: "High-throughput fluorogenic assay for determination of botulinum type B neurotoxin protease activity." ANALYTICAL BIOCHEMISTRY, vol. 291, no. 2, 15 avril 2001 (2001-04-15), pages 253-261, XP001023970 ISSN: 0003-2697**
- **FAWZI AHMAD B ET AL: "A rapid and selective endothelin-converting enzyme assay: Characterization of a phosphoramidon-sensitive enzyme from guinea pig lung membrane." ANALYTICAL BIOCHEMISTRY, vol. 222, no. 2, 1994, pages 342-350, XP002168333 ISSN: 0003-2697 cité dans la demande**
- **SOLEILHAC ET AL: "A sensitive and rapid fluorescence-based assay for determination of tetanus toxin peptidase activity" ANALYTICAL BIOCHEMISTRY,ACADEMIC PRESS, SAN DIEGO, CA,US, vol. 241, 1996, pages 120-127, XP002160418 ISSN: 0003-2697 cité dans la demande**
- **TAKAHASHI MASAAKI ET AL: "Purification and characterization of endothelin-converting enzyme from rat lung." JOURNAL OF BIOLOGICAL CHEMISTRY, vol. 268, no. 28, 1993, pages 21394-21398, XP002168334 ISSN: 0021-9258 cité dans la demande**
- **XU DONG ET AL: "ECE-1: A membrane-bound metalloprotease that catalyzes the proteolytic activation of big endothelin-1." CELL, vol. 78, no. 3, 1994, pages 473-485, XP002168335 ISSN: 0092-8674 cité dans la demande**

**EP 1 268 850 B1**

**Description**

La présente invention concerne d'une manière générale l'utilisation d'un reste PyA-(Z)$_x$-pNF dans des substrats de peptidases ou protéases à des fins d'identification et/ou de caractérisation de composés capables d'inhiber celles-çi. Elle vise notamment l'utilisation d'un tel reste et plus particulièrement le reste Pya-pNF dans une séquence pour doser l'enzyme de conversion de l'endothéline et/ou identifier des inhibiteurs de cette enzyme.

Le contrôle de la pression artérielle est sous la dépendance de plusieurs peptides qui possèdent soit des actions de vasoconstriction des vaisseaux (c'est le cas en particulier de l'angiotensine II, des endothélines, de l'urotensine), soit des actions vasodilatatrices (c'est le cas en particulier de la bradykinine ou du peptide atrial natriurétique qui joue de plus un rôle sur l'élimination d'eau et de chlorure de sodium).

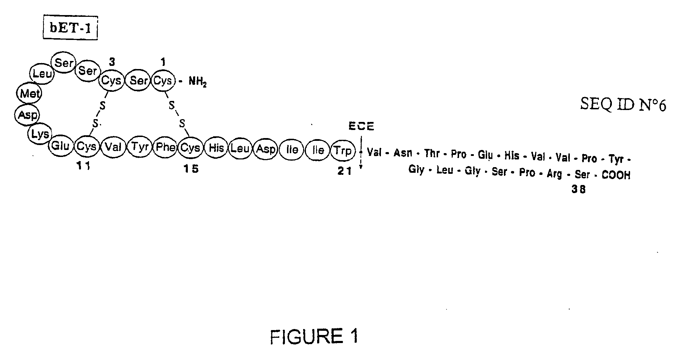

Parmi tous ces peptides, l'endothéline-1 (ET-1) qui est un peptide possédant une partie cyclique, assurée par deux ponts disulfure, et une courte partie linéaire (Figure 1) est considérée comme un très puissant vasoconstricteur par action sur les muscles lisses des parois des vaisseaux, en particulier au niveau cardiaque (artères coronaires).

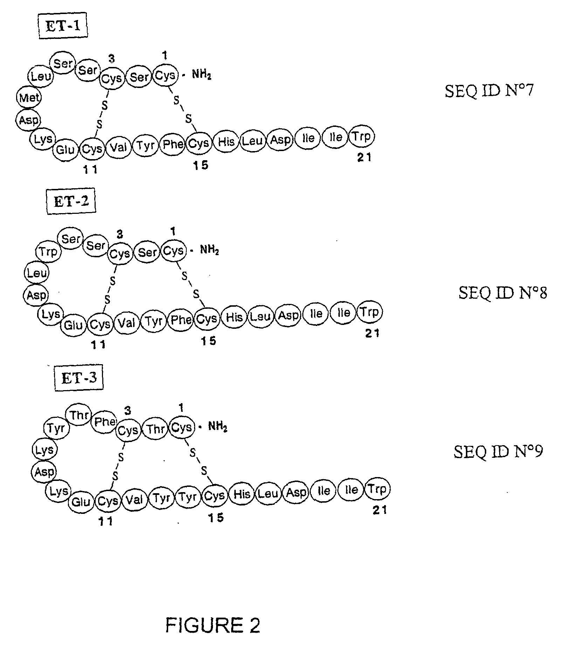

L'endothéline-1 fait partie d'une famille d'isopeptides où l'on trouve l'endothéline-2 (ET-2) et l'endothéline-3 (ET-3) qui ne diffèrent de l'ET-1 que par quelques changements ponctuels d'acides aminés (Figures 2).

Les endothélines sont sécrétées par les cellules endothéliales sous forme de précurseurs inactifs, les "big-endothélines", qui sont clivées par des métallopeptidases à zinc dénommées enzyme de conversion de l'endothéline (ECE) pour donner naissance aux peptides actifs. Ainsi, si on prend l'exemple de l'ET-1, l'ECE-1 hydrolyse la liaison Trp$^{21}$-Val$^{22}$ de la big-endothéline-1 qui comporte 38 acides aminés pour générer deux fragments dont l'un, N-terminal, constitue l'endothéline-1 active. Dans le cas de l'ET-3, c'est la liaison Trp$^{21}$-Ile$^{22}$ qui est clivée.

La big-ET-1 et l'ET-1 sont détectées dans le plasma et l'ECE est une enzyme cytosolique ou membranaire caractérisée dans ce cas par un court fragment intracellulaire suivi d'une hélice transmembranaire d'environ 25 acides aminés et d'un domaine extracellulaire très important qui contient le site actif. L'enzyme est constituée par deux sous-unités identiques reliées par un pont disulfure (*Shimada et al., Biochem. J. (1996) 315, 863-867).*

L'administration de faibles doses de big-endothéline provoque une forte augmentation de la pression artérielle démontrant le clivage du précurseur avec formation d'endothéline. Cette réponse peut être bloquée par un antagoniste du récepteur à l'ET-1.

Il existe donc un intérêt considérable dans l'inhibition de l'ECE par des composés dont les applications sont potentiellement très nombreuses dans les affections cardiovasculaires. On peut citer en particulier le traitement de l'infarctus du myocarde, de l'angor, des spasmes vasculaires consécutifs à une hémorragie cérébrale, des spasmes des vaisseaux périphériques, de l'hypertension pulmonaire, de l'insuffisance cardiaque et/ou rénale, de troubles liés au diabète, de l'asthme, de la prévention des resténoses, des fibroses cardiaques et vasculaires.

En l'espèce, certaines pathologies cardiovasculaires pourraient par conséquent être prévenues ou au moins réduites par une diminution de la concentration circulante d'endothélines en inhibant l'enzyme qui leur donne naissance, c'est-à-dire l'ECE.

Toutefois, la recherche intensive de tels inhibiteurs exige de disposer d'un test d'identification simple et robotisable, de manière à permettre la réalisation d'un grand nombre d'essais en un temps réduit.

Or, les tests actuels sont soit difficiles, longs, peu spécifiques et/ou onéreux.

Les tests, où les deux fragments produits par action de l'ECE sur la big-ET sont dosés par HPLC, ont pour inconvénient majeur de nécessiter de grandes quantités d'enzyme et de big-ET.

Le test basé sur le clivage de la big-ET marquée à l'iode 125 et donnant naissance à la $^{125}$I ET-1 et sa détermination par fixation sur son récepteur n'est pas utilisable pour des essais à haut-flux *(Fawzi, A.B.* ; *Clemen, R.M. & Wright, D.L.* (1994) *Anal. Biochem. 222, 342-350).* Il en est de même de tests cellulaires mesurant l'augmentation de GMPc induite par la fixation d'ET-1 sur son récepteur.

Les dosages radioimmunologiques RIA nécessitent pour leur part une série très complexe d'expériences successives, sont relativement peu sélectifs et exigent l'utilisation, à la fois de big-ET et de $^{125}$I ET-1. Ces dosages complexes ne se prêtent pas au tri à haut-flux de très grandes séries d'inhibiteurs potentiels avec robotisation du test et demandent la synthèse ou l'achat d'endothéline iodée radioactive, dont le temps d'utilisation est limité.

Plus récemment, il a été proposé un test basé sur l'utilisation d'un substrat radioactif obtenu par fixation sur la partie N-terminale de peptides de 21 acides aminés contenant le site de clivage Trp-Val d'un reste propionyl tritié (FR n° 96 02673). Le métabolite N-terminal radioactif formé est extrait par un solvant organique sous agitation et un échantillon de ce solvant est prélevé, mélangé à un liquide de scintillation puis la radioactivité mesurée à l'aide d'un compteur. La succession d'étapes ne permet pas là encore de robotiser l'essai et de plus exige l'utilisation d'un radioélément et donc la synthèse périodique du substrat tritié.

Fitzgerald et al (Anal Biochem, 254, 226-230, 1997) divulgue l'utilisation d'un substrat à fluorescence éteinte contenant Ab2 (aminobenzoic acid) et pNF, séparés de 2 acides aminés, pour la détection de la protéase du virus d'immunodéficience féline (FIV).

La présente invention a précisément pour objet de proposer un nouveau test de dosage permettant de s'af-

3

franchir des inconvénients rencontrés avec les tests évoqués précédemment.

La présente invention repose plus particulièrement sur la mise en évidence par les inventeurs que l'utilisation de la pyrenylalanine (PyA) associée à l'acide modifié L-paranitrophénylalanine (pNF) était particulièrement intéressante à des fins de diagnostic.

La pyrenylalanine est un acide aminé synthétique qui possède une capacité de fluorescence importante. Avantageusement, cette fluorescence du reste PyA est quasi totalement éteinte lorsque PyA est placé à proximité et de préférence accolé au reste pNF.

En conséquence, cette fluorescence naturelle du PyA ne peut se manifester qu'à partir du moment où ce reste n'est plus soumis à l'effet répresseur du reste pNF, par exemple lorsqu'il en est physiquement séparé, notamment par clivage par une protéase ou peptidase quelle que soit la classe (métallopeptidase, serine protéase, aspartyl-protéase, cystéine protéase) à laquelle elle appartient.

Un reste de type PyA-pNF apparaît donc comme un outil particulièrement intéressant lorsqu'il est introduit au niveau d'un substrat et plus particulièrement d'une séquence peptidique ou pseudopeptidique, pour visualiser par fluorescence la manifestation d'un clivage.

Un premier aspect de l'invention concerne donc l'utilisation d'un reste

$$PyA\text{-}(Z)_x\text{-}pNF,$$

avec Z représentant un enchaînement comprenant jusqu'à 4 acides aminés de série L, naturels ou non et x représentant 1 ou 0, dans un substrat à des fins de détection, identification et/ou dosage par fluorescence d'une protéase ou peptidase susceptible de cliver la ou une liaison établie entre PyA et pNF et/ou de composés à activité inhibitrice ou activatrice vis-à-vis d'une protease ou peptidase capable de cliver cette liaison.

L'utilisation revendiquée est tout particulièrement intéressante pour détecter, identifier et/ou doser par fluorescence des composés de type peptidases ou protéases quelque soit le groupe auquel elles appartiennent, c'est-à-dire des sérine protéases, cystéines protéases, aspartyl protéases et métalloprotéases.

La présente invention est basée sur l'apparition d'une fluorescence provoquée par la séparation de PyA et pNF par clivage d'une liaison établie entre eux.

Z comprend jusqu'à 4 acides aminés identiques ou différents de préférence choisis dans la liste des 20 acides aminés naturels. La longueur de l'enchaînement d'acides aminés figurée par Z est en fait à ajuster en fonction de la spécificité de substrat de la peptidase et de manière à ce que la distance séparant PyA et pNF soit compatible avec la manifestation de l'effet répresseur de pNF sur la fluorescence naturellement émise par PyA.

Un second critère pour cet ajustement est lié à la nécessité de préserver la spécificité et l'affinité du composé clivant naturellement le substrat incluant PyA-$(Z)_x$-pNF. Le choix du reste PyA-$(Z)_x$-pNF est par conséquent effectué en prenant en compte ces deux critères.

Le substrat dans lequel est introduit le reste peptidyle PyA-$(Z)_x$-pNF est peptidique.

Un mode de réalisation particulier de l'invention concerne l'inclusion d'un reste PyA-$(Z)_x$-pNF et de préférence PyA-pNF dans les séquences peptidiques reconnues par l'ECE. On peut ainsi identifier, détecter et/ou doser l'ECE ou des inhibiteurs ou activateurs de l'ECE. L'hydrolyse du reste PyA-pNF produit une très intense augmentation de fluorescence (environ de 150 à 800 fois pour une dégradation de 5% du substrat).

Selon un mode de réalisation préféré de l'invention, le reste dipeptidyle PyA-pNF est présent, dans la séquence peptidique reconnue par l'ECE, au niveau du site de clivage naturel auquel il se substitue.

De manière inattendue, le reste dipeptidyle PyA-pNF se révèle clivable par l'ECE et n'altère pas l'affinité et la spécificité de cette enzyme vis-à-vis de la séquence peptidique qui l'inclue.

Dans le cas particulier où la séquence peptidique correspond à celle de la big-endothéline-1 ou 2, la liaison substituée est la liaison Trp[21]-Val[22] et de la big-endothéline-3, Trp[21]-Ile[22].

La big-ET-1 (19-35) utilisée comme produit de départ peut être préparée selon les méthodes usuelles telles que notamment par un synthétiseur automatique en phase solide tel que le modèle, par exemple, 431A de Applied Biosystems utilisant la technologie Fmoc, tel que décrit dans la référence *ATHERTON, E. et SHEPPARD, R.C.* (1989) *Solid Phase Peptide Synthesis : a practical approach, IRL Press, Oxford.*

L'extrême sensibilité de l'utilisation revendiquée permet d'opérer dans des volumes très faibles, de l'ordre de 50 à 100 $\mu$l, avec des concentrations réduites en substrat de l'ordre de 2 $\mu$M à 50 $\mu$M.

Un second aspect de l'invention concerne des produits de formule I :

$$Ac\text{-}(X_1)_n\ PyA\text{-}(Z)_x\text{-}pNF\text{-}(X'_1)_m\text{-}R \qquad (I)$$

dans laquelle

-  les symboles $X_1$, et $X'_1$ représentent des acides aminés,

- Z représente un enchaînement d'acides aminés de série L, naturels ou non et x représente 1 ou 0,
- R représente un groupement OH ou $NH_2$,
- Ac représente un groupement acétyle,
- n est un entier variant de 2 à 6 et m de 0 à 16 et de préférence de 3 à 13.

Le test de la présente invention est ainsi basé sur l'utilisation de peptides de formule (I) dont la partie N-terminale est acylée ce qui présente au moins deux avantages : protéger du clivage par les aminopeptidases et renforcer l'hydrophobicité du fragment N-terminal qui sera généré par l'hydrolyse.

Cette manifestation de fluorescence est liée à la génération dans le clivage d'un métabolite de formule générale II :

$$Ac\text{-}(X_1)_n\text{-}PyA\text{-}(Z')_x \qquad \text{(II)}$$

dans laquelle
- $X_1$ et n sont tels que définis précédemment,
- Z' représente un ou plusieurs acides aminés de série L, naturels ou non et
- x représente 1 ou 0.

Plus préférentiellement, x représente 0 dans les formules (I) et (II).

Selon une première variante de l'invention, les composés de formule (I) répondent à la formule générale (IA)

Ac-(Z1)-(Z2)-Ile-Ile-PyA-pNF-AsN-Thr-Pro-Glu-His-Val-Val-Pro-Tyr-Gly-Leu-Gly-

Ser-(Z4)-(Z5)-(Z6)-COOH          (SEQ ID N°1) (IA)

dans laquelle $Z_1$, $Z_2$, $Z_4$, $Z_5$ et $Z_6$, identiques ou différents, représentent un acide aminé.

Plus particulièrement, les composés de formules I ou IA sont tels que :

- $Z_1$ et $Z_2$ sont tels que :

    soit $Z_1$ et $Z_2$ représentent une simple liaison,
    soit $Z_1$ représente une simple liaison et $Z_2$ représente l'acide aminé Asp,
    soit $Z_1$ et $Z_2$ représentent respectivement les acides aminés Leu et Asp,

- $Z_4$, $Z_5$ et $Z_6$ sont tels que :

    soit $Z_4$, $Z_5$ et $Z_6$ représentent une simple liaison,
    soit $Z_5$ et $Z_6$ représentent une simple liaison et $Z_4$ représente l'acide aminé Pro,
    soit $Z_6$ représente une simple liaison et $Z_4$ et $Z_5$ représentent respectivement les acides aminés Pro et Arg,
    soit $Z_4$, $Z_5$ et $Z_6$ représentent respectivement les acides aminés Pro, Arg et Ser.

Sont notamment couverts par cette formule générale (IA) des substrats reconnus par l'ECE.

A titre représentatif des substrats reconnus par l'ECE et conformes à la formule générale I on peut tout particulièrement citer :

Ac-Ile-Ile-Pya-pNF-Asn-Thr-Pro-Glu-His-Val-Val-Pro-Tyr-Gly-Leu-Gly-Ser-COOH

(SEQ ID N°2)

Ac-Arg-Pro-Lys-Pro-Gln-Gln-Pya-pNF-Gly-Leu-Met-$NH_2$ (SEQ ID N°3)

ou

Ac-Ser-Gly-PyA-Lys-Ala-Phe-Ala-pNF-Gly-Lys $NH_2$ (SEQ ID N°8).

La SEQ ID N°2 est par ailleurs conforme à la formule générale IA

De même, à titre représentatif des métabolites de formule II, on peut notamment mentionner :

<div align="center">

Ac-Ile-Ile-PyA (SEQ ID N°4)

</div>

<div align="center">

Ac-Arg-Pro-Lys-Gln-Gln-PyA (SEQ ID N°5)

</div>

ou

<div align="center">

Ac-Ser-Gly-PyA-Lys-Ala (SEQ ID N°9)

</div>

Selon une seconde variante de l'invention, les composés de formule générale I répondent à la formule générale (IB)

<div align="center">

Ac-Ser-Lys-Gly-Pya-$(Z)_x$-pNF -Gly-Gly-Lys-$NH_2$                (IB)

(SEQ ID N°6)

</div>

avec Z et x étant tels que définis en formule générale I

Selon cette variante particulière, le métabolite généré répond à la formule générale IIa

<div align="center">

Ac-Ser-Lys-Gly-Pya-$(Z')_x$

(SEQ ID N°7)

</div>

avec Z' et x tels que définis en formule générale II.

A titre illustratif des substrats conformes à la formule IB de l'invention on peut notamment citer :

. comme substrat de la kallikréine en tant que modèle d'une enzyme de la famille serine protéase

<div align="center">

Ac-Ser-Lys-Gly-Pya-Lys-Ile-pNF-Gly-Gly-Lys-$NH_2$ (SEQ ID n° 10)

(Métabolite formé: Ac-Ser-Lys-Gly-Pya-Lys (SEQ ID N° 11))

</div>

. comme substrat de la papaïne en tant que modèle d'une enzyme de la famille cystéine protéase

<div align="center">

Ac-Ser-Lys-Gly-Pya-Leu-Lys-pNF-Gly-Gly-Lys-$NH_2$ (SEQ ID N° 12)

(Métabolite formé: Ac-Ser-Lys-Gly-Pya-Leu (SEQ ID N° 13))

</div>

. comme substrat de la pepsine en tant que modèle d'une enzyme de la famille aspartyl protéase

Ac-Ser-Lys-Gly-Pya-Leu-pNF-Gly-Gly-Lys-NH$_2$ (SEQ ID N° 14)

(Métabolite formé: Ac-Ser-Lys-Gly-Pya-Leu (SEQ ID N° 13))

. comme substrat de la néprilysine en tant que modèle d'une enzyme de la famille métallo endo peptidase

Ac-Ser-Lys-Gly-Pya-Lys-Phe-pNF-Gly-Gly-Lys-NH$_2$ (SEQ ID N° 15)

(Métabolite formé: Ac-Ser-Lys-Gly-Pya-Lys (SEQ ID N° 11))

. comme substrat d'ACE en tant que modèle d'une enzyme de la famille Métallo di peptidyl carboxy peptidase)

Ac-Ser-Lys-Gly-Pya-Ala-Gly-Phe-pNF-OH (SEQ ID N° 16)

(Métabolite formé: Ac-Ser-Lys-Gly-Pya-Ala-Gly (SEQ ID N° 17))

La préparation des composés conformes à la présente invention relève des compétences de l'homme de l'art.

Les composés revendiqués peuvent être obtenus par les méthodes usuelles de synthèse en phase solide selon la méthode de Merrifield sur un synthétiseur automatique comme par exemple l'appareil 431A de Applied Biosystems. La chimie utilisée correspond à la technologie Fmoc et la protection des chaînes latérales permettant leur clivage par l'acide trifluoroacétique tel que décrit par *E. Atherlon et R.C. Sheppard (1989) dans "Solid Phase Peptide Synthesis : a practical approach, IRL Press, Oxford ".*

La réaction d'acylation pour conduire aux composés de formule I peut être réalisée dans les conditions usuelles connues de l'homme du métier.

La L-pyrenylalanine est obtenue selon un procédé de synthèse asymétrique décrit dans la publication *Soleilhac, J.M. et al. Anal. Biochem.* (1996) *241, 120-127.* La pureté des peptides finaux est estimée supérieure à 99% par HPLC en phase inverse et l'identité des peptides par spectrométrie de masse electrospray.

Les couplages sont effectués selon des techniques conventionnelles à l'aide d'agent de couplage comme HAUT, PyBrop et de préférence en utilisant dicyclohexylcarbodiimide / hydroxybenzotriazole.

Un autre aspect de la présente invention concerne l'utilisation d'un composé de formule générale I tel que défini ci-dessus pour la détection, l'identification et/ou le dosage d'une protéase choisie parmi la famille des Sérine protéases, Cystéine protéases, Aspartyl protéases, et métallopeptidases et plus préférentiellement l'ECE et l'étude de composés capables d'inhiber ou d'activer ladite enzyme.

Selon une variante préférée de l'invention, le composé à doser est soit l'ECE soit un composé capable d'inhiber ou d'activer l'enzyme de conversion de l'endothéline.

La présente invention a également pour objet un procédé pour détecter, identifier et/ou doser un composé capable d'inhiber ou d'activer l'enzyme de conversion de l'endothéline, caractérisé en ce qu'il comprend :

- la mise en présence en solution d'un composé de formule générale I, et reconnue par l'enzyme de conversion de l'endothéline avec ladite enzyme de conversion de l'endothéline, et au moins un composé susceptible d'inhiber ou activer l'ECE,
- la mesure de la fluorescence émise en présence, et le cas échéant en absence, du composé à détecter, identifier et/ou doser et
- en ce que l'absence ou une diminution de fluorescence indique la présence d'un composé inhibiteur de l'enzyme de conversion de l'endothéline.

En fait, l'ordre de mise en présence des trois composés n'est pas déterminant. On peut tout d'abord mettre en présence le composé de formule générale I avec l'ECE puis introduire en une étape ultérieure le présumé inhibiteur ou activateur.

Selon une autre variante, le composé de formule générale I est d'abord mis en présence du composé à doser puis l'ECE.

Le composé tel que défini ci-dessus auquel on applique le test défini ci-dessus pour déterminer son activité inhibitrice de l'ECE peut être de nature diverse, sans limitation, et par exemple, peut être choisi parmi les phosphonimides, les phosphamides et les dérivés de ces produits, ou encore les inhibiteurs de métalloprotéinases tels que les dérivés thiol, carboxylates ou hydroxamates.

On peut également citer comme composé le phosphoramidon et le N-(phényl-éthylphosphonyl)-Leu-Trp (TAKEDA), dont les activités inhibitrices de l'ECE sont connues.

Le composé à tester peut se présenter sous une forme isolée, être connu ou non et/ou être présent dans une banque de composés, un extrait biologique ou de l'eau.

Selon une variante préférée, le substrat est :

Ac-Ile-Ile-Pya-pNF-Asn-Thr-Pro-Glu-His-Val-Val-Pro-Tyr-Gly-Leu-Gly-Ser-COOH

(SEQ ID N°2)(peptide 1s)

Ac-Arg-Pro-Lys-Pro-Gln-Gln-Pya-pNF-Gly-Leu-Met-NH$_2$ (SEQ ID N°3)

(peptide 2s).

ou

Ac-Ser-Gly-PyA-Lys-Ala-Phe-Ala-pNF-Gly-Lys NH$_2$

(SEQ ID N°8) (peptide 3s)

L'addition de doses croissantes d'un composé, capable d'inhiber l'activité enzymatique de l'ECE vis à vis du substrat de formule générale I par exemple, se traduira par une diminution de l'intensité de la fluorescence due au métabolite, formé par l'ECE, de formule Ac-Ile-Ile-PyA, (peptide 1m), Ac-Arg-Pro-Lys-Gln-Gln-PyA (SEQ ID N°5) (peptide 2m) ou Ac-Ser-Gly-PyA-Lys-Ala (SEQ ID N°9) (peptide 3m) dont la fluorescence n'est pas modifiée durant plusieurs heures. La mesure de cette intensité rapportée à une courbe étalon établie par mélanges du substrat et du métabolite fluorescent formé permet donc soit d'évaluer le pouvoir inhibiteur en pourcentage d'inhibition à une concentration donnée fixe du produit P, soit de déterminer précisément son $IC_{50}$ puis son $K_I$ à l'aide du $K_m$ du substrat et de plusieurs concentrations du produit P.

L'extrême sensibilité du dosage permet d'opérer dans des volumes très faibles (100 $\mu$l) avec des concentrations de l'ordre de 20 $\mu$M du substrat 1 s (soit 2 nmole correspondant à 4.43 $\mu$g par essai) ou 5 $\mu$M du substrat 2s. Le test est donc très facilement utilisable en plaques à 96 puits ou plus, permettant sa robotisation avec détermination automatique des $K_I$, lorsque le fluorimètre de lecture est relié à un ordinateur possédant un logiciel commercial approprié.

Incubés avec l'ECE, les peptides 1s, 2s et 3s ne conduisent qu'à deux métabolites comme le montre l'analyse de la réaction par HPLC et correspondant au clivage unique au niveau de la liaison peptidique PyA-pNF.

Avantageusement, ces substrats sont extrêmement sélectifs puisque aucun clivage n'est observé par HPLC après incubation durant 2 h à 37°C avec des métallopeptidases à zinc purifiées appartenant à la même famille que l'ECE, telles que par exemple la néprilysine (NEP) ou l'enzyme de conversion de l'angiotensine (ACE).

Enfin, la sensibilité et la spécificité du test est telle que l'on peut utiliser aussi bien des ECE purifiées *(Takahashi et al. J. Biol. Chem. (1993) 268, 21395-21398)*, qu'une préparation membranaire de cellules CHO dans lesquelles l'ECE a été transfectée *(Xu et al. Cell* (1994) 78, *473-485)* ou même une préparation membranaire d'un tissu animal riche en ECE (poumon ou cerveau de rat). La séquence et la structure de l'ECE étant très conservées d'une espèce à l'autre, c'est en particulier le cas du rat et de l'homme, les résultats ($K_I$ d'inhibiteurs par exemple), obtenus sur des ECE de rongeurs purifiées ou non, sont identiques à ceux obtenus sur l'enzyme humaine recombinante.

La présente invention propose donc un test d'identification d'inhibiteurs de l'ECE ainsi que la détermination de leur pouvoirs inhibiteurs par un essai fluorimétrique, très rapide, reproductible et permettant des tests robotisables et très nombreux pouvant donc s'adapter à une sélection à haut débit de molécules inhibitrices.

La présente invention propose également la production de produits industriels nouveaux, éventuellement utilisables sous forme de kits comprenant des plaques de 96, 192 et 384 puits prêts à l'emploi et contenant soit l'ECE lyophilisée, soit un substrat tel que défini dans la formule générale I et illustré dans le cas de 1s, 2s et 3s, auxquels on ajoute, éventuellement par utilisation d'un robot, les réactifs nécessaires à la détermination des pouvoirs inhibiteurs de séries de molécules.

Un autre aspect de l'invention concerne une méthode de diagnostic d'une pathologie liée à un taux plasmatique anormal en enzyme de conversion de l'endothéline, caractérisé en ce qu'elle met en oeuvre un composé de formule I tel que défini précédemment.

Les figures et exemples ci-après sont présentés à titre illustratif et non limitatif de la présente invention.

Figure 1 : séquence peptidique de la big-endothéline.

Figure 2 : séquences peptidiques endothélines ET-1, ET-2 et ET-3.

Figure 3 : graphes représentant le spectre d'émission fluorescente du peptide 1s et de son métabolite 1 m (figure 3a) et du peptide 2s et de son métabolite 2m (figure 3b).

## EXEMPLE 1

### PREPARATION DES PEPTIDES.

Les peptides identifiés ci-après 1s et 2s et les métabolites fluorescents correspondants contenant le reste pyrenylalanine, sont préparés en phase solide par la méthode de Merrifield sur un synthétiseur automatique en utilisant la stratégie Fmoc et la protection des chaînes latérales généralement sous forme de reste t-butyl, ether ou ester, trityl ou Boc comme déjà décrit dans *"Solid Phase Peptide Synthesis : A practical approach IRL Press Oxford "*.

Les couplages sont effectués par utilisation de dicyclohexylcarbodiimide/hydroxybenzotriazole dans la N-méthylpyrrolidone. Le peptide final est obtenu après clivage et déprotection des chaînes latérales par l'acide trifluoroacétique. La solution est évaporée sous vide, le peptide précipité par l'éther et purifié par HPLC sur une colonne Vydac $C_{18}$.

Les peptides sont analysés par spectrométrie de masse et RMN $^1$H (400 ou 600 MHz).

Composé 1s

Ac-Ile-Ile-Pya-pNF-Asn-Thr-Pro-Glu-His-Val-Val-Pro-Tyr-Gly-Leu-Gly-Ser-COOH (SEQ ID N°2)

$MH^+_{(exp)}$ : 2101.1 ; $MH^+_{(theo)}$ : 2102.7

Composé 2s

Ac-Arg-Pro-Lys-Pro-Gln-Gln-Pya-pNF-Gly-Leu-Met-$NH_2$ (SEQ ID N°3)

$MH^+_{(exp)}$ : 1558.8 ; $MH^+_{(theo)}$ : 1559.7

Ces peptides peuvent se conserver après lyophilisation sous forme de poudre, à l'abri de la lumière et à 4°C ou à -20°C pendant plus de 6 mois sans altération. De même les solutions mères des substrats ($10^{-3}$-$10^{-4}$ M) peuvent être conservées à 4°C et à l'abri de la lumière durant plusieurs semaines.

## EXEMPLE 2

### 2.1 : Matériels et méthodes.

On peut utiliser diverses préparations contenant l'ECE comme par exemple l'ECE-1c humaine recombinante exprimée dans des cellules Cos-1 *(Shimada et al., J. Biol. Chem. (1994) 269, 18275-18278)* ou CHO *(Xu et al. Cell (1994) 78, 473-485)*.

Dans tous les cas, la présence de l'enzyme est testée par Western blot à l'aide d'un anticorps et sa concentration évaluée par comparaison avec l'enzyme purifiée.

L'activité ECE est déterminée dans des plaques à 96 puits généralement dans les conditions suivantes :

100 $\mu$l de tampon 50 mM Tris-maleate, pH 6.5, 20 $\mu$M ou 5 $\mu$M de peptide fluorescent 1s ou 2s et 10 $\mu$l d'une solution de ECE diluée en fonction de sa pureté et de manière à ce que le clivage du substrat fluorescent reste inférieur à 5%. On incube de 20 minutes à 1 h selon le substrat utilisé de 20°C à 37°C, en présence ou en absence d'un inhibiteur potentiel en concentrations fixes (par exemple $5 \times 10^{-6}$ M et $10^{-6}$ M) ou à des concentrations croissantes de $10^{-11}$ à $10^{-5}$ M pour déterminer un $K_I$. La réaction est stoppée de diverses manières, par exemple chauffage à 90°C, refroidissement brutal à 4°C, en addition d'un solvant organique comme le dioxane à 20% du volume final puis la lecture est effectuée à l'aide d'un fluorimètre ($\lambda_{ex}$ = 340 nm ; $\lambda_{em}$ = 400 nm) éventuellement couplé au système d'analyse robotisé. L'utilisation d'un fluorimètre à réseau accroît encore considérablement la sensibilité du dosage par lecture de la fluorescence à 377 nm. Les contrôles sont effectués en présence de tous les réactifs mais sans l'ECE ou avec l'ECE préalablement inactivée par chauffage à 90°C, 5 minutes. Les $K_m$ des substrats (constante d'affinité de l'enzyme pour un substrat) calculés par utilisation du programme ENZFITTER sont :

$$1s, Km = 22.1 \pm 0.9 \ \mu M \ ;$$

$$2s, Km = 2 \pm 0.5 \ \mu M.$$

Les valeurs de $K_I$ des inhibiteurs peuvent être déterminées automatiquement à partir des pourcentages de dégradation à différentes concentrations et comparaison avec une gamme étalon. La fluorescence observée fournit directement après linéarisation, une valeur de $IC_{50}$, elle-même transformée en $K_I$ à partir de la relation de Cheng-Prussof, $K_I = IC_{50}/1 + ([S]/K_m)$, avec [S] étant la concentration en substrat.

**2.2: Dosage de l'activité ECE vis-à-vis des peptides fluorescents 1s et 2s en absence d'inhibiteurs.**

La fluorescence des substrats 1s et 2s ainsi que celle de leurs métabolites a été appréciée en absence d'inhibiteurs. Le protocole retenu est celui décrit précédemment.

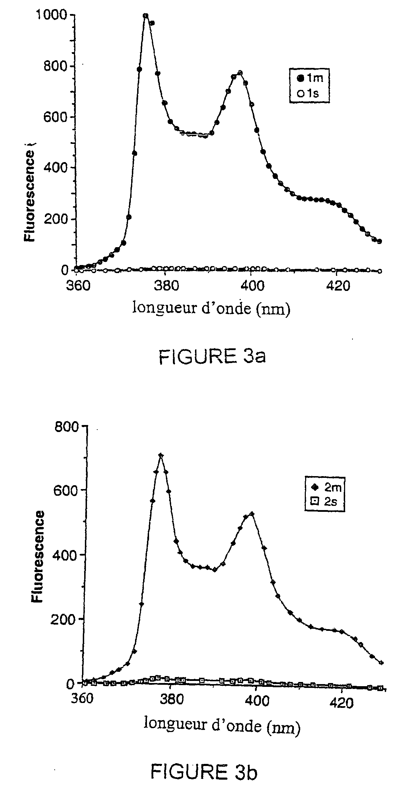

Les figures 3a et 3b rendent compte respectivement des spectres d'émission de fluorescence du peptide 1s et de son métabolite 1 m et du peptide 2s et de son métabolite 2 m.
En ce qui concerne les produits formés, leur analyse a été effectuée pour s'assurer de la sélectivité du substrat par HPLC sur colonne nucléosyl $C_{18}$, 7 $\mu$m/300 Å (4.6 x 70 mm) en utilisant un tampon B (0.038% de TFA dans $CH_3CN/H_2O$, 9/1 en vol.) et un gradient de 40-47% de tampon B en 10 minutes (débit 1 ml/min).
Dans tous les cas, un seul site de clivage au niveau du dipeptide L.PyA-Lp.NF a été observé, générant uniquement deux métabolites.

## EXEMPLE 3

En reproduisant le protocole décrit en exemple 2, il a été procédé à l'évaluation du comportement inhibiteur de plusieurs composés.
Les composés testés sont :

|  |  |
|---|---|
| Témoins négatifs | R = Rétrothiorphan |
|  | T = Thiorphan |
|  | C = Captopril |
|  | L = Lysinopril |
| Témoin positif | P = Phosphoramidon |

Les conditions spécifiques retenues pour les essais figurent ci-après :

|  | Blanc * | Témoin | Test |
|---|---|---|---|
| Tris maléate pH 6.5 | 90 $\mu$l | 80 $\mu$l | 70 $\mu$l |
| Inhibiteur | / | / | 10 $\mu$l |

Suite de tableau

|  | Blanc * | Témoin | Test |
|---|---|---|---|
| Enzyme | / | 10 μl | 10 μl |
| * L'utilisation d'enzyme, dénaturée par HCl 6N ou par chauffage à 80°C, 10 minutes, ne modifie pas la fluorescence résiduelle du blanc. | | | |

On préincube 10 minutes à 37°C. Le substrat ECE est ensuite rajouté à raison de 10°C. L'ensemble est incubé entre 20 minutes et 1 heure à 37°C et la réaction ensuite stoppée soit par refroidissement ou ajout de dioxane (20 μl).
La lecture sur cytofluor est réalisée à 4°C avec les spécificités suivantes : Excitation 340 nm, Emission 400 nm et avec un Gain 85.
Les essais sont réalisés sur une microplaque de 96 puits.
Le tableau I rend compte pour chaque puit de la nature du composé testé ainsi que de sa concentration. Les composés ont été testés à deux concentrations.

**TABLEAU I**

| 1 | 2 | 3 | 4 | 5 | 6 | 7 | 8 | 9 | 10 | 11 | 12 |
|---|---|---|---|---|---|---|---|---|----|----|----|
| Blanc | | Témoin | | R $10^{-6}$ M | | R $5\times10^{-6}$ M | | T $10^{-6}$ M | | T $5\times10^{-6}$ M | |
| C $10^{-6}$ M | | C $5\times10^{-6}$ M | | L $10^{-6}$ M | | L $5\times10^{-6}$ M | | P $5\times10^{-6}$ M | | P $10^{-6}$ M | |

## EXEMPLE 4

En tableau II figure l'intensité de fluorescence mesurée pour chaque puits. Une valeur de fluorescence faible, témoigne d'une activité inhibitrice forte du composé testé. Le comportement inhibiteur du composé P est parfaitement vérifié.

**TABLEAU II**

| 1 | 2 | 3 | 4 | 5 | 6 | 7 | 8 | 9 | 10 | 11 | 12 |
|------|------|------|------|------|------|------|------|------|------|------|------|
| 67 | 68 | 1081 | 1094 | 1124 | 1106 | 1043 | 1077 | 1124 | 1127 | 1109 | 1121 |
| 1086 | 1089 | 1128 | 1121 | 1117 | 1093 | 1095 | 1119 | 140 | 133 | 455 | 459 |

**Revendications**

1. Utilisation d'un reste PyA-$(Z)_x$-pNF,
avec Z représentant un enchaînement comprenant jusqu'à 4 acides aminés de série L, naturels ou non, et x représentant 1 ou 0, dans un substrat à des fins de détection, identification et/ou dosage par fluorescence d'une protéase ou peptidase susceptible de cliver la ou une liaison établie entre PyA et pNF et/ou de composés à activité inhibitrice ou activatrice vis-à-vis d'une protéase ou peptidase capable de cliver cette liaison.

2. Utilisation selon la revendication 1 **caractérisée en ce que** la protéase est choisie parmi les familles des sérine protéases, cystéines protéases, aspartyl protéases et métalloprotéases.

3. Utilisation selon la revendication 2, **caractérisée en ce que** la protéase est une métalloprotéase.

4. Utilisation selon l'une des revendications 1 à 3, **caractérisée en ce que** le substrat est une séquence peptidique reconnue par l'enzyme de conversion de l'endothéline (ECE) et **en ce que** l'on détecte, identifie et/ou dose l'ECE ou des inhibiteurs de l'ECE.

5. Utilisation selon l'une des revendications 1 à 4, **caractérisée en ce que** dans le reste peptidyle PyA-$(Z)_x$-pNF, x représente 0.

6. Utilisation selon l'une des revendications 4 ou 5, **caractérisée en ce que** le dipeptide PyA-pNF est présent dans la séquence peptidique reconnue par l'ECE au niveau du site de clivage naturel auquel il se substitue.

**7.** Utilisation selon la revendication 6, **caractérisée en ce que** la liaison substituée est la liaison Trp$^{21}$-Val$^{22}$ de la big-endothéline-1 ou big-endothéline-2.

**8.** Utilisation selon la revendication 6, **caractérisée en ce que** la liaison substituée est la liaison Trp$^{21}$-Ile$^{22}$ de la big-endothéline 3.

**9.** Composé de formule (I) :

$$Ac(X_1)_n \, PyA-(Z)_x\text{-}pNF\text{-}(X'_1)_m\text{-}R \qquad (I)$$

dans laquelle :

- X$_1$ et X'$_1$ représentent indépendamment l'un de l'autre un acide aminé,
- Z représente un enchaînement comprenant jusqu'à 4 acides aminés de série L, naturels ou non et x représente 1 ou 0,
- R représente OH ou NH$_2$,
- Ac un groupement acétyle et
- n un entier variant de 2 à 6 et m de 0 à 16.

**10.** Composé selon la revendication 9, **caractérisé en ce que** x est égal à 0.

**11.** Composé selon la revendication 9 ou 10, de formule (Ia) :

Ac-(Z1)-(Z2)-Ile-Ile-PyA-pNF-AsN-Thr-Pro-Glu-His-Val-Val-Pro-Tyr-Gly-Leu-Gly-Ser-(Z4)-(Z5)-(Z6)-COOH     (Ia)    (SEQ ID N°1)

dans laquelle Z$_1$, Z$_2$, Z$_4$, Z$_5$ et Z$_6$, identiques ou différents, représentent un acide aminé.

**12.** Composé selon la revendication 11, **caractérisé en ce que**

- Z$_1$ et Z$_2$ sont tels que :

    soit Z$_1$ et Z$_2$ représentent une simple liaison,
    soit Z$_1$ représente une simple liaison et Z$_2$ représente l'acide aminé Asp,
    soit Z$_1$ et Z$_2$ représentent respectivement les acides aminés Leu et Asp,

- Z$_4$, Z$_5$ et Z$_6$ sont tels que :

    soit Z$_4$, Z$_5$ et Z$_6$ représentent une simple liaison,
    soit Z$_5$ et Z$_6$ représentent une simple liaison et Z$_4$ représente l'acide aminé Pro,
    soit Z$_6$ représente une simple liaison et Z$_4$ et Z$_5$ représentent respectivement les acides aminés Pro et Arg,
    soit Z$_4$, Z$_5$ et Z$_6$ représentent respectivement les acides aminés Pro, Arg et Ser.

**13.** Composé selon l'une des revendications 10 à 12, **caractérisé en ce qu**'il s'agit de :

Ac-Ile-Ile-Pya-pNF-Asn-Thr-Pro-Glu-His-Val-Val-Pro-Tyr-Gly-Leu-Gly-Ser-COOH (SEQ ID N°2)

Ac-Arg-Pro-Lys-Pro-Gln-Gln-Pya-pNF-Gly-Leu-Met-NH$_2$ (SEQ ID N°3)

ou

Ac-Ser-Gly-PyA-Lys-Ala-Phe-Ala-pNF-Gly-Lys NH$_2$ (SEQ ID N°8).

14. Composé selon la revendication 9, **caractérisé en ce qu'**il répond à la formule (IB) :

Ac-Ser-Lys-Gly-Pya-(Z)$_x$-pNF -Gly-Gly-Lys-NH$_2$ (SEQ ID N°6)

dans laquelle

- Z représente un enchaînement comprenant jusqu'à 4 acides aminés de série L, naturels ou non et x représente 1 ou 0.

15. Composé selon la revendication 14, **caractérisé en ce qu'**il s'agit de :

Ac-Ser-Lys-Gly-Pya-Lys-Ile-pNF-Gly-Gly-Lys-NH$_2$ (SEQ ID N° 10)

Ac-Ser-Lys-Gly-Pya-Leu-Lys-pNF-Gly-Gly-Lys-NH$_2$ (SEQ ID N° 12)

Ac-Ser-Lys-Gly-Pya-Leu-pNF-Gly-Gly-Lys-NH$_2$ (SEQ ID N° 14)

Ac-Ser-Lys-Gly-Pya-Lys-Phe-pNF-Gly-Gly-Lys-NH$_2$ (SEQ ID N° 15)

ou

Ac-Ser-Lys-Gly-Pya-Ala-Gly-Phe-pNF-OH (SEQ ID N° 16).

16. Composé de formule générale II :

Ac(X$_1$)$_n$PyA(Z')$_x$ (II)

dans laquelle

- X$_1$, identiques ou différents, représentent un acide aminé et
- n un entier variant de 2 à 6 et
- x représente 0 ou 1, et
- Z' représente un enchaînement comprenant jusqu'à 4 acides aminés de série L, naturels ou non.

17. Composé selon la revendication 16, **caractérisé en ce qu'**il s'agit de :

Ac-Ile-Ile-PyA (SEQ ID N°4)

Ac-Arg-Pro-Lys-Gln-Gln-PyA (SEQ ID N°5)

Ac-Ser-Lys-Gly-Pya-Lys (SEQ ID N° 11)

Ac-Ser-Lys-Gly-Pya-Leu (SEQ ID N° 13)

Ac-Ser-Lys-Gly-Pya-Ala-Gly (SEQ ID N° 17).

**18.** Utilisation d'un composé tel que défini en revendications 9 à 15 pour la détection, l'identification et/ou le dosage d'une protéase choisie parmi la famille des sérine protéases, cystéines protéases, aspartyl protéases et métalloprotéases ou d'un composé capable d'inhiber ou d'activer ladite enzyme.

**19.** Utilisation selon la revendication 18 pour la détection, l'identification et/ou le dosage de métalloprotéases.

**20.** Procédé pour détecter, identifier et/ou doser un composé capable d'inhiber ou d'activer l'enzyme de conversion de l'endothéline (ECE), **caractérisé en ce qu'**il comprend :

- la mise en présence en solution d'un composé de formule générale I, tel que défini en revendications 9 à 15 et reconnu par l'enzyme de conversion de l'endothéline avec ladite enzyme de conversion de l'endothéline et au moins un composé susceptible d'inhiber ou activer l'ECE,
- la mesure de la fluorescence émise en présence, et le cas échéant en absence, du composé à détecter, identifier et/ou doser et
- **en ce que** l'absence ou une diminution de fluorescence indique la présence d'un composé inhibiteur de l'enzyme de conversion de l'endothéline.

**21.** Procédé selon la revendication 20, **caractérisé en ce que** le composé de formule générale I est :

Ac-Ile-Ile-Pya-pNF-Asn-Thr-Pro-Glu-His-Val-Val-Pro-Tyr-Gly-Leu-Gly-Ser-COOH (SEQ ID N° 2)

Ac-Arg-Pro-Lys-Pro-Gln-Gln-Pya-pNF-Gly-Leu-Met-NH$_2$ (SEQ ID N° 3)

ou

Ac-Ser-Gly-PyA-Lys-Ala-Phe-Ala-pNF-Gly-Lys NH$_2$ (SEQ ID N°8).

**22.** Méthode de diagnostic d'une pathologie liée à un taux plasmatique anormal en enzyme de conversion de l'endothéline, **caractérisé en ce qu'**elle met en oeuvre un composé selon l'une des revendications 9 à 15.

**Patentansprüche**

**1.** Verwendung eines PyA-(Z)x-pNF-Restes, wobei Z eine Kette, die bis zu 4 gegebenenfalls nicht natürliche Aminosäuren der L-Reihe umfasst, und x 1 oder 0 bedeutet, in einem Substrat für die Detektion, Identifizierung und/oder quantitative Analyse einer Protease oder Peptidase, die in der Lage ist, die oder eine Bindung zwischen PyA und pNF zu spalten, und/oder einer Verbindung mit inhibierender oder aktivierender Wirkung auf eine Protease oder Peptidase, die in der Lage ist, diese Bindung zu spalten, durch Fluoreszenz.

**2.** Verwendung nach Anspruch 1, **dadurch gekennzeichnet, dass** die Protease aus den Familien der Serinproteasen, Cysteinproteasen, Aspartylproteasen und Metallproteasen ausgewählt wird.

**3.** Verwendung nach Anspruch 2, **dadurch gekennzeichnet, dass** die Protease eine Metallprotease ist.

**4.** Verwendung nach einem der Ansprüche 1 bis 3, **dadurch gekennzeichnet, dass** das Substrat eine Peptidsequenz ist, die von dem Endothelin-Konversions-Enzym (ECE) erkannt wird, und dass das ECE oder ein Inhibitor des ECE detektiert, identifiziert und/oder quantitativ analysiert wird.

**5.** Verwendung nach einem der Ansprüche 1 bis 4, **dadurch gekennzeichnet, dass** in dem Peptidylrest PyA-(Z)x-pNF x 0 bedeutet.

**6.** Verwendung nach Anspruch 4 oder 5, **dadurch gekennzeichnet, dass** das Dipeptid PyA-pNF in der Peptidsequenz vorhanden ist, die von dem ECE an der natürlichen Spaltungsstelle, an welcher dieses sich substituiert, erkannt wird.

**7.** Verwendung nach Anspruch 6, **dadurch gekennzeichnet, dass** die substituierte Bindung die Trp21-Val22-Bindung von big-Endothelin-1 oder big-Endothelin-2 ist.

**8.** Verwendung nach Anspruch 6, **dadurch gekennzeichnet, dass** die substituierte Bindung die Trp21-Ile22-Bindung von big-Endothelin 3 ist.

**9.** Verbindung mit der Formel (I):

$$Ac(X1)nPyA-(Z)x-pNF-(X'1)m-R \qquad (I),$$

in welcher:

- X1 und X'1 unabhängig voneinander eine Aminosäure bedeuten,
- Z eine Kette, die bis zu 4 gegebenenfalls nicht natürliche Aminosäuren der L-Reihe umfasst, und x 1 oder 0,
- R OH oder NH2,
- Ac eine Acetylgruppe und
- n eine ganze Zahl von 2 bis 6 und m 0 bis 16

bedeutet.

**10.** Verbindung nach Anspruch 9, **dadurch gekennzeichnet, dass** x gleich 0 ist.

**11.** Verbindung nach Anspruch 9 oder 10 mit der Formel (Ia) :

```
Ac-(Z1)-(Z2)-Ile-Ile-PyA-pNF-Asn-Thr-Pro-Glu-His-Val-
Val-Pro-Tyr-Gly-Leu-Gly-Ser-(Z4)-(Z5)-(Z6)-COOH (Ia)
(SEQ ID Nr. 1),
```

in welcher Z1, Z2, Z4, Z5 und Z6, die gegebenenfalls voneinander verschieden sind, eine Aminosäure bedeuten.

**12.** Verbindung nach Anspruch 11, **dadurch gekennzeichnet, dass**

- Z1 und Z2 derart sind, dass:

    • Z1 und Z2 jeweils eine Einfachbindung bedeuten,
    • Z1 eine Einfachbindung und Z2 die Aminosäure Asp bedeutet oder
    • Z1 und Z2 jeweils die Aminosäuren Leu und Asp bedeuten und

- Z4, Z5 und Z6 derart sind, dass:

    • Z4, Z5 und Z6 jeweils eine Einfachbindung bedeuten,
    • Z5 und Z6 eine Einfachbindung bedeuten und Z4 die Aminosäure Pro bedeutet,
    • Z6 eine Einfachbindung bedeutet und Z4 und Z5 jeweils die Aminosäuren Pro und Arg bedeuten oder
    • Z4, Z5 und Z6 jeweils die Aminosäuren Pro, Arg und Ser bedeuten.

**13.** Verbindung nach einem der Ansprüche 10 bis 12, **dadurch gekennzeichnet, dass** es sich handelt um:

```
Ac-Ile-Ile-Pya-pNF-Asn-Thr-Pro-Glu-His-Val-Val-Pro-
Tyr-Gly-Leu-Gly-Ser-COOH (SEQ ID Nr. 2),
```

```
Ac-Arg-Pro-Lys-Pro-Gln-Pya-pNF-Gly-Leu-Met-NH2 (SEQ
ID Nr. 3)
```

oder

```
Ac-Ser-Gly-PyA-Lys-Ala-Phe-Ala-pNF-Gly-Lys-NH2 (SEQ
ID Nr. 8).
```

**14.** Verbindung nach Anspruch 9, **dadurch gekennzeichnet, dass** sie der Formel (IB) entspricht:

```
Ac-Ser-Lys-Gly-Pya-(Z)x-pNF-Gly-Gly-Lys-NH2 (SEQ ID
Nr. 6),
```

in welcher

- Z eine Kette, die bis zu 4 gegebenenfalls nicht natürliche Aminosäuren der L-Reihe umfasst, und x 1 oder 0 bedeutet.

**15.** Verbindung nach Anspruch 14, **dadurch gekennzeichnet, dass** es sich handelt um:

```
Ac-Ser-Lys-Gly-Pya-Lys-Ile-pNF-Gly-Gly-Lys-NH2 (SEQ
ID Nr. 10)
```

```
Ac-Ser-Lys-Gly-Pya-Leu-Lys-pNF-Gly-Gly-Lys-NH2 (SEQ
ID Nr. 12)
```

```
Ac-Ser-Lys-Gly-Pya-Leu-pNF-Gly-Gly-Lys-NH2 (SEQ ID
Nr. 14)
```

```
Ac-Ser-Lys-Gly-Pya-Lys-Phe-pNF-Gly-Gly-Lys-NH2 (SEQ
ID Nr. 15) oder
```

```
Ac-Ser-Lys-Gly-Pya-Ala-Gly-Phe-pNF-OH (SEQ ID Nr.
16).
```

**16.** Verbindung mit der allgemeine Formel II:

Ac(X1)nPyA(Z')x          (II),

in welcher

- X1 gegebenenfalls voneinander verschieden sind und eine Aminosäure bedeuten und
- n eine ganze Zahl von 2 bis 6,
- x 0 oder 1 und
- Z' eine Kette, die bis zu 4 gegebenenfalls nicht natürliche Aminosäuren der L-Reihe umfasst,

bedeutet.

**17.** Verbindung nach Anspruch 16, **dadurch gekennzeichnet, dass** es sich handelt um:

```
Ac-Ile-Ile-PyA (SEQ ID Nr. 4)


Ac-Arg-Pro-Lys-Gln-Gln-PyA (SEQ ID Nr. 5)


Ac-Ser-Lys-Gly-Pya-Lys (SEQ ID Nr. 11)


Ac-Ser-Lys-Gly-Pya-Leu (SEQ ID Nr. 13)



Ac-Ser-Lys-Gly-Pya-Ala-Gly (SEQ ID Nr. 17).
```

**18.** Verwendung einer wie in den Ansprüchen 9 bis 15 definierten Verbindung zur Detektion, Identifizierung und/oder quantitativen Analyse einer Protease, die aus der Familie der Serinproteasen, Cysteinproteasen, Aspartylproteasen und Metallproteasen ausgewählt ist, oder einer Verbindung, die in der Lage ist, dieses Enzym zu inhibieren oder zu aktivieren.

**19.** Verwendung nach Anspruch 18 zur Detektion, Identifizierung und/oder quantitativen Analyse von Metallproteasen.

**20.** Verfahren zur Detektion, Identifizierung und/oder quantitativen Analyse einer Verbindung, die in der Lage ist, das Endothelin-Konversions-Enzym (ECE)zu inhibieren oder zu aktivieren, **dadurch gekennzeichnet, dass** es das:

- In-Berührung-Bringen einer Verbindung mit der allgemeinen Formel I, wie in den Ansprüchen 9 bis 15 definiert, die von dem Endothelin-Konversions-Enzym erkannt wird, mit dem Endothelin-Konversions-Enzym und mit mindestens einer Verbindung, die in der Lage ist, das ECE zu inhibieren oder zu aktivieren, in Lösung, und
- Messen der Fluoreszenz, die bei Anwesenheit und gegebenenfalls bei Abwesenheit der zu detektierenden, zu identifizierenden und/oder quantitativ zu analysierenden Verbindung emittiert wird, umfasst, und dass die
- Abwesenheit oder eine Verringerung der Fluoreszenz das Vorhandensein einer das Endothelin-Konversions-Enzym inhibierenden Verbindung anzeigt.

**21.** Verfahren nach Anspruch 20, **dadurch gekennzeichnet, dass** die Verbindung mit der allgemeinen Formel I:

```
Ac-Ile-Ile-Pya-pNF-Asn-Thr-Pro-Glu-His-Val-Val-Pro-
Tyr-Gly-Leu-Gly-Ser-COOH (SEQ ID Nr. 2)
```

```
Ac-Arg-Pro-Lys-Pro-Gln-Gln-Pya-pNF-Gly-Leu-Met-NH2
 (SEQ ID Nr. 3)
```

oder

```
Ac-Ser-Gly-PyA-Lys-Ala-Phe-Ala-pNF-Gly-Lys-NH2 (SEQ
ID Nr. 8)
```

ist.

22. Verfahren zur Diagnose einer Krankheit, die mit einem anormalen Plasmagehalt an Endothelin-Konversions-Enzym verbunden ist, **dadurch gekennzeichnet, dass** in ihm eine Verbindung nach einem der Ansprüche 9 bis 15 verwendet wird.

## Claims

1. Use of a PyA-$(Z)_x$-pNF residue,
   where Z represents a chain comprising up to 4 amino acids of the L series, which may or may not be natural, and x represents 1 or 0, in a substrate for the purposes of detecting, identifying and/or assaying by fluorescence a protease or peptidase capable of cleaving the bond or one of the bonds established between PyA and pNF, and/or compounds with inhibitory or activating action on a protease or peptidase capable of cleaving this bond.

2. Use according to Claim 1, **characterized in that** the protease is chosen from the serine protease, cysteine protease, aspartyl protease and metalloprotease families.

3. Use according to Claim 2, **characterized in that** the protease is a metalloprotease.

4. Use according to any one of Claims 1 to 3, **characterized in that** the substrate is a peptide sequence recognized by the endothelin converting enzyme (ECE) and **in that** the ECE or inhibitors of the ECE are detected, identified and/or assayed.

5. Use according to any one of Claims 1 to 4, **characterized in that**, in the peptidyl residue PyA-$(Z)_x$-pNF, x represents 0.

6. Use according to either one of Claims 4 and 5, **characterized in that** the dipeptide PyA-Pnf is present in the peptide sequence recognized by the ECE at the natural cleavage site for which it is substituted.

7. Use according to Claim 6, **characterized in that** the substituted bond is the $Trp^{21}$-$Val^{22}$ bond of big endothelin-1 or big endothelin-2.

8. Use according to Claim 6, **characterized in that** the substituted bond is the $Trp^{21}$-$Ile^{22}$ bond of big endothelin-3.

9. Compound of formula (I):

$$Ac(X_1)_n PyA-(Z)_x-pNF-(X'_1)_m-R \qquad (I)$$

in which:

- each of $X_1$ and $X'_1$ represents, independently of each other, an amino acid,
- Z represents a chain comprising up to 4 amino acids of the L series, which may or may not be natural, and x represents 1 or 0,
- R represents OH or $NH_2$,
- Ac represents an acetyl group, and
- n is an integer ranging from 2 to 6 and m ranges from 0 to 16.

**10.** Compound according to Claim 9, **characterized in that** x is equal to 0.

**11.** Compound according to Claim 9 or 10, of formula (Ia):

```
Ac-(Z1)-(Z2)-Ile-Ile-PyA-pNF-AsN-Thr-Pro-Glu-His-Val-Val-
Pro-Tyr-Gly-Leu-Gly-Ser-(Z4)-(Z5)-(Z6)-COOH   (Ia) (SEQ ID
No. 1)
```

in which each of $Z_1$, $Z_2$, $Z_4$, $Z_5$ and $Z_6$, which may be identical or different, represents an amino acid.

**12.** Compound according to Claim 11, **characterized in that**:

- $Z_1$ and $Z_2$ are such that :

either each of $Z_1$ and $Z_2$ represents a single bond,
or $Z_1$ represents a single bond and $Z_2$ represents the amino acid Asp, or $Z_1$ and $Z_2$ represent, respectively, the amino acids Leu and Asp,

- $Z_4$, $Z_5$ and $Z_6$ are such that:

either each of $Z_4$, $Z_5$ and $Z_6$ represents a single bond,
or each of $Z_5$ and $Z_6$ represents a single bond and $Z_4$ represents the amino acid Pro,
or $Z_6$ represents a single bond and $Z_4$ and $Z_5$ represent, respectively, the amino acids Pro and Arg,
or $Z_4$, $Z_5$ and $Z_6$ represent, respectively, the amino acids Pro, Arg and Ser.

**13.** Compound according to any one of Claims 10 to 12, **characterized in that** it is:

```
Ac-Ile-Ile-Pya-pNF-Asn-Thr-Pro-Glu-His-Val-Val-Pro-Tyr-Gly-
                   Leu-Gly-Ser-COOH    (SEQ ID No. 2)
```

```
Ac-Arg-Pro-Lys-Pro-Gln-Gln-Pya-pNF-Gly-Leu-Met-NH₂      (SEQ
                          ID No. 3)
```

or

```
Ac-Ser-Gly-PyA-Lys-Ala-Phe-Ala-pNF-Gly-Lys NH₂     (SEQ ID
                          No. 8)
```

**14.** Compound according to Claim 9, **characterized in that** it corresponds to formula (IB):

```
Ac-Ser-Lys-Gly-Pya-(Z)ₓ-pNF-Gly-Gly-Lys-NH₂   (SEQ ID No. 6)
```

in which

- Z represents a chain comprising up to 4 amino acids of the L series, which may or may not be natural, and x represents 1 or 0.

**15.** Compound according to Claim 14, **characterized in that** it is:

Ac-Ser-Lys-Gly-Pya-Lys-Ile-pNF-Gly-Gly-Lys-NH$_2$    (SEQ ID No. 10)

Ac-Ser-Lys-Gly-Pya-Leu-Lys-pNF-Gly-Gly-Lys-NH$_2$    (SEQ ID No. 12)

Ac-Ser-Lys-Gly-Pya-Leu-pNF-Gly-Gly-Lys-NH$_2$    (SEQ ID No. 14)

Ac-Ser-Lys-Gly-Pya-Lys-Phe-pNF-Gly-Gly-Lys-NH$_2$    (SEQ ID No. 15) or

Ac-Ser-Lys-Gly-Pya-Ala-Gly-Phe-pNF-OH    (SEQ ID No. 16)

16. Compound of general formula II:

$$AC(X_1)_nPYA(Z')_x \qquad (II)$$

in which

- each of the identical or different $X_1$ represents an amino acid, and
- n represents an integer ranging from 2 to 6, and
- x represents 0 or 1, and
- Z' represents a chain comprising up to 4 amino acids of the L series, which may or may not be natural.

17. Compound according to Claim 16, **characterized in that** it is:

Ac-Ile-Ile-PyA (SEQ ID No. 4)

Ac-Arg-Pro-Lys-Gln-Gln-PyA    (SEQ ID No. 5)

Ac-Ser-Lys-Gly-Pya-Lys    (SEQ ID No. 11)

Ac-Ser-Lys-Gly-Pya-Leu    (SEQ ID No. 13)

Ac-Ser-Lys-Gly-Pya-Ala-Gly    (SEQ ID No. 17).

18. Use of a compound as defined in Claims 9 to 15 for detecting, identifying and/or assaying a protease selected from the serine protease, cysteine protease, aspartyl protease and metalloprotease family, or a compound capable of inhibiting or activating the said enzyme.

19. Use according to Claim 18 for detecting, identifying and/or assaying metalloproteases.

20. Method for detecting, identifying and/or assaying a compound capable of inhibiting or activating the endothelin converting enzyme (ECE), **characterized in that** it comprises:

- bringing a compound of general formula I, as defined in Claims 9 to 15 and recognized by the endothelin converting enzyme, into contact in solution with the said endothelin converting enzyme and at least one compound capable of inhibiting or activating the ECE,
- measuring the fluorescence emitted in the presence, and if appropriate in the absence, of the compound to be detected, identified and/or assayed, and
- **in that** the absence or decrease of fluorescence indicates the presence of a compound inhibiting the endothelin converting enzyme.

21. Method according to Claim 20, **characterized in that** the compound of general formula I is:

```
Ac-Ile-Ile-Pya-pNF-Asn-Thr-Pro-Glu-His-Val-Val-Pro-Tyr-Gly-
                Leu-Gly-Ser-COOH     (SEQ ID No. 2)


        Ac-Arg-Pro-Lys-Pro-Gln-Gln-Pya-pNF-Gly-Leu-Met-NH₂ (SEQ ID
                            No. 3)
```

or

```
        Ac-Ser-Gly-PyA-Lys-Ala-Phe-Ala-pNF-Gly-Lys NH₂     (SEQ ID
                            No. 8).
```

22. Method of diagnosing a pathology related to an abnormal plasma level of endothelin converting enzyme, **characterized in that** it uses a compound according to any one of Claims 9 to 15.

bET-1

SEQ ID N°6

FIGURE 1

ET-1

SEQ ID N°7

ET-2

SEQ ID N°8

ET-3

SEQ ID N°9

FIGURE 2

FIGURE 3a

FIGURE 3b

LISTE DE SEQUENCES

<110> INSERM

<120> Utilisation d'un reste PyA-(Z)x-pNF à des fins de détection, identification et/ou dosage par fluorescence.

<130> BET01/0363

<140>
<141>

<160> 17

<170> PatentIn Ver. 2.1

<210> 1
<211> 22
<212> PRT
<213> Séquence artificielle

<220>
<223> Description de la séquence artificielle:substrat peptidique, Xaa en position 1 = Z1 ; Xaa en position 2 Z2 Xaa en position 5 PyA ; Xaa en position 6 = pNF ; Xaa en position 20 = Z4 ; Xaa en position 21 = Z5 ; Xaa en position 22 = Z6.

<400> 1

```
    Xaa Xaa Ile Ile Xaa Xaa Asn Thr Pro Glu His Val Val Pro Tyr Gly
     1               5                  10                  15

    Leu Gly Ser Xaa Xaa Xaa
                  20
```

<210> 2
<211> 16
<212> PRT
<213> Séquence artificielle

<220>
<223> Description de la séquence artificielle:substrat peptidique, Xaa en position 3 = pNF.

<400> 2

```
    Ile Ile Xaa Asn Thr Pro Glu His Val Val Pro Tyr Gly Leu Gly Ser
     1               5                  10                  15
```

<210> 3
<211> 11
<212> PRT
<213> Séquence artificielle

<220>
<223> Description de la séquence artificielle:substrat peptidique, Xaa en position 7 = PyA ; Xaa en position 8 = pNF.

<400> 3

Arg Pro Lys Pro Gln Gln Xaa Xaa Gly Leu Met

<210> 4
<211> 3
<212> PRT
<213> Séquence artificielle

<220>
<223> Description de la séquence artificielle:substrat peptidique, Xaa en position 3 = PyA.

<400> 4

Ile Ile Xaa

<210> 5
<211> 6
<212> PRT
<213> Séquence artificielle

<220>
<223> Description de la séquence artificielle:substrat peptidique, Xaa en position 6 = PyA.

<400> 5

Arg Pro Lys Gln Gln Xaa

<210> 6
<211> 9
<212> PRT
<213> Séquence artificielle

<220>
<223> Description de la séquence artificielle:substrat peptidique, Xaa en position 4 = PyA ; Xaa en position 5 = Z; Xaa en position 7 = pNF

<400> 6

Ser Lys Gly Xaa Xaa Xaa Gly Gly Lys

<210> 7
<211> 5
<212> PRT
<213> Séquence artificielle

<220>
<223> Description de la séquence artificielle:substrat peptidique, Xaa en position 4 = PyA ; Xaa en position 5 = Z'.

<400> 7

```
                    Ser Lys Gly Xaa Xaa
                     1                 5
```

<210> 8
<211> 10
<212> PRT
<213> Séquence artificielle

<220>
<223> Description de la séquence artificielle:substrat peptidique, Xaa en position 3 = PyA ; Xaa en position 9 = pNF.

<400> 8

```
              Ser Gly Xaa Lys Ala Phe Ala Xaa Gly Lys
               1               5                    10
```

<210> 9
<211> 5
<212> PRT
<213> Séquence artificielle

<220>
<223> Description de la séquence artificielle:substrat peptidique, Xaa en position 3 = PyA.

<400> 9

```
                    Ser Gly Xaa Lys Ala
                     1               5
```

<210> 10
<211> 10
<212> PRT
<213> Séquence artificielle

<220>
<223> Description de la séquence artificielle:substrat peptidique, Xaa en position 4 = PyA ; Xaa en position 7 = pNF.

<400> 10

```
              Ser Lys Gly Xaa Lys Ile Xaa Gly Gly Lys
                1               5                  10
```

<210> 11
<211> 5
<212> PRT
<213> Séquence artificielle

<220>
<223> Description de la séquence artificielle:substrat peptidique, Xaa en position 4 = PyA.

<400> 11

```
                         Ser Lys Gly Xaa Lys
                          1                 5
```

<210> 12
<211> 10
<212> PRT
<213> Séquence artificielle

<220>
<223> Description de la séquence artificielle:substrat peptidique, Xaa en position 4 = PyA ; Xaa en position 7 = pNF.

<400> 12

```
              Ser Lys Gly Xaa Leu Lys Xaa Gly Gly Lys
               1               5                   10
```

<210> 13
<211> 5
<212> PRT
<213> Séquence artificielle

<220>
<223> Description de la séquence artificielle:substrat peptidique, Xaa en position 4 = PyA.

<400> 13

```
                         Ser Lys Gly Xaa Leu
                         1 5
```

<210> 14
<211> 9
<212> PRT
<213> Séquence artificielle

<220>
<223> Description de la séquence artificielle:substrat peptidique, Xaa en position 4 = PyA ; Xaa en position 8 = pNF.

<400> 14

```
              Ser Lys Gly Xaa Leu Xaa Gly Gly Lys
               1               5
```

<210> 15
<211> 10
<212> PRT
<213> Séquence artificielle

<220>
<223> Description de la séquence artificielle:substrat peptidique, Xaa en position 4 = PyA ; Xaa en position 7 = pNF.

<400> 15

```
            Ser Lys Gly Xaa Lys Phe Xaa Gly Gly Lys
             1               5                   10
```

<210> 16
<211> 8
<212> PRT
<213> Séquence artificielle

<220>
<223> Description de la séquence artificielle:substrat peptidique, Xaa en position 4 = PyA ; Xaa en position 8 = pNF.

<400> 16

```
              Ser Lys Gly Xaa Ala Gly Phe Xaa
               1               5
```

<210> 17
<211> 6
<212> PRT
<213> Séquence artificielle

<220>
<223> Description de la séquence artificielle:substrat peptidique, Xaa en position 4 = PyA.

<400> 17

```
                Ser Lys Gly Xaa Ala Gly
                 1               5
```